# FASCICULE DE BREVET EUROPEEN

(11) **EP 0 685 225 B1**
(45) Date de publication et mention de la délivrance du brevet: **30.06.1999**
(21) Numéro de dépôt: 95401059.1
(22) Date de dépôt: 05.05.1995
(51) Int. Cl.: A61K 7/42

(54) **Compositions cosmétiques antisolaires et utilisations**
Kosmetische Sonnenschutzmittel und Verwendungen
Cosmetic sunscreening compositions and uses

(30) Priorité: 03.06.1994 FR 9406833
(43) Date de publication de la demande: 06.12.1995
(73) Titulaire: L'OREAL, 75008 Paris (FR)
(72) Inventeur: Hansenne, Isabelle, F-75017 Paris (FR); van Leeuwen, Alexandra Victoria, F-75020 Paris (FR)
(74) Mandataire: Andral, Christophe André Louis

(56) Documents cités:
- EP-A- 0 005 182
- EP-A- 0 518 772
- WO-A-91/11989

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association d'au moins deux filtres lipophiles particuliers, l'un des filtres possédant des propriétés solubilisantes vis à vis de l'autre filtre. L'invention concerne par ailleurs un procédé de solubilisation d'un ou deux filtres lipophiles solides particuliers au moyen d'un autre filtre lipophile liquide spécifique.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Pour diverses raisons liées en particulier à un meilleur confort d'utilisation (douceur, émollience, facilité d'application et autres), les compositions antisolaires actuelles se présentent le plus souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques capables d'absorber selectivement les rayonnements UV nocifs, ces filtres étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV). Selon leur caractère lipophile ou au contraire hydrophile, ces filtres peuvent se répartir, respectivement, soit dans la phase grasse, soit dans la phase aqueuse, de la composition finale.

Il s'avère qu'un filtre particulièrement intéressant et largement utilisé à ce jour est constitué par le 4-méthylbenzylidène camphre, qui est notamment vendu sous la dénomination commerciale de "EUSOLEX 6300" par la Société Merck.

Il s'agit d'un filtre lipophile, fortement actif dans l'UV-B, mais qui présente la particularité et aussi le désavantage d'être solide à température ambiante. De ce fait, son utilisation dans une composition cosmétique antisolaire implique certaines contraintes au niveau de sa formulation et de sa mise en oeuvre, en particulier lorsqu'il s'agit de trouver des solvants permettant de le solubiliser correctement. A cet égard, on fait aujourd'hui le plus souvent appel à des huiles comme des esters et plus particulièrement des benzoates d'alkyles en C₁₂-C₁₅ ("FINSOLV TN" de chez Finetex), ou à des triglycérides et notamment des triglycérides d'acides gras en C₈-C₁₂ ("MIGLYOL 812" de chez Hüls), ou encore à des monoalcools ou des polyols tels que l'éthanol, ainsi qu'à leurs mélanges. Ces produits, bien que possédant des propriétés solubilisantes vis à vis du filtre susmentionné, présentent néanmoins pour inconvénient de n'avoir aucune activité propre dans le domaine de la filtration du rayonnement UV, tant UV-A qu'UV-B.

Or, la Demanderesse vient maintenant de découvrir, de façon inattendue et surprenante, que l'α-cyano-β, β-diphénylacrylate de 2-éthylhéxyle, encore appelé octocrylène, constitue un solvant particulièrement remarquable pour le 4-méthylbenzylidène camphre, ce dernier composé présentant en effet dans l'octocrylène des solubilités extrêmement élevées et en tous cas nettement supérieures à celles obtenues avec tous les autres solvants usuels utilisés à ce jour, ce qui permet, à quantité égale de solvant, de mettre en oeuvre des quantités plus importantes de filtre. On notera que l'octocrylène est un filtre lipophile liquide déja connu pour son activité dans l'UV-B, mais ses propriétés solubilisantes vis à vis du premier filtre cité n'ont, quant à elles, jamais été décrites. L'intérêt de la présente découverte est ainsi double, en ce sens qu'il est maintenant possible d'une part d'effectuer la solubilisation du 4-méthylbenzylidène camphre par un solvant autre que ceux antérieurement connus, ce qui est toujours intéressant en soi, et ceci, d'autre part, tout en réalisant une augmentation substantielle, à concentration égale de ce filtre dans la composition antisolaire finale, du niveau de protection attachée à cette dernière. Par ailleurs, il a été également trouvé que l'octocrylène présente un excellent pouvoir solubilisant vis à vis d'un mélange particulier de deux filtres lipophiles solides, à savoir un mélange entre le filtre UV-B précédemment mentionné (4-méthylbenzylidène camphre) et un filtre actif dans l'UV-A qui est constitué par le 4-(ter.-butyl) 4'-méthoxy dibenzoylméthane, ce qui rend possible l'obtention de formulations antisolaires offrant une protection maximale dans toute la gamme des UV nocifs (280 nm -400 nm), ces dernières compositions présentant par ailleurs une parfaite stabilité.

Toutes ces découvertes sont à la base de la présente invention.

Ainsi, conformément à l'un des objets de la présente invention, il est maintenant proposé de nouvelles compositions cosmétiques, en particulier antisolaires, qui sont caractérisées par le fait qu'elles comprennent, dans un support cosmétiquement acceptable, (i) du 4-méthylbenzylidène camphre sous forme solubilisée à titre de filtre actif dans l'UV-B, et (ii) de l'α-cyano-β, β-diphénylacrylate de 2-éthylhéxyle en une quantité suffisante pour solubiliser à lui seul l'ensemble dudit filtre UV-B.

Selon un autre aspect de la présente invention, les compositions ci-dessus contiennent en outre, également sous une forme solubilisée, un filtre actif dans l'UV-A et qui est constitué par le 4-(ter.-butyl) 4'-méthoxy dibenzoylméthane.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

La présente invention a également enfin pour objet l'utilisation de l'α-cyano-β, β-diphénylacrylate de 2-éthylhéxyle pour solubiliser du 4-méthylbenzylidène camphre, éventuellement en association avec du 4-(ter.-butyl) 4'-méthoxy dibenzoylméthane, contenu(s) dans une composition cosmétique antisolaire.

D'autres caractéristiques, aspects et avantages de la présente invention apparaitront à la lecture de la description détaillée qui va suivre.

Comme indiqué précédemment, le 4-méthylbenzylidène camphre (composé A essentiel) est un filtre connu en soi, actif dans l'UV-B et se présentant sous une forme solide, qui est vendu notamment sous la dénomination commerciale de "EUSOLEX 6300" par la Société MERCK ou de "PARSOL 5000" par la Société GIVAUDAN. Ce produit répond à la formule (I) suivante :

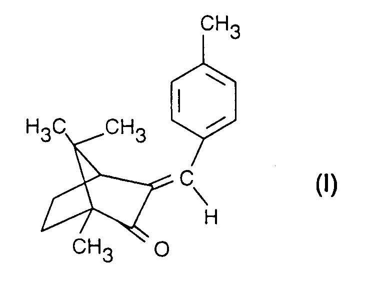

De même, l'α-cyano-β, β-diphénylacrylate de 2-éthylhéxyle, connu aussi sous le nom d'octocrylène (composé B essentiel), est également un produit disponible commercialement, et est vendu notamment sous la dénomination de "UVINUL N 539" par la Société BASF. Il répond à la formule (II) suivante : 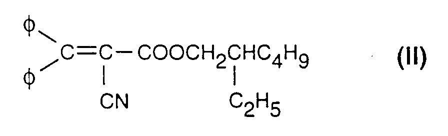 dans laquelle φ désigne un radical phényle.

Enfin, le 4-(ter.-butyl) 4'-méthoxy dibenzoylméthane (composé C optionnel), qui est un filtre actif dans l'UV-A connu en soi pouvant rentrer de façon avantageuse dans les compositions conformes à l'invention, est, pour sa part, notamment proposé à la vente sous la dénomination commerciale de "PARSOL 1789" par la Société GIVAUDAN, et répond à la formule (III) :

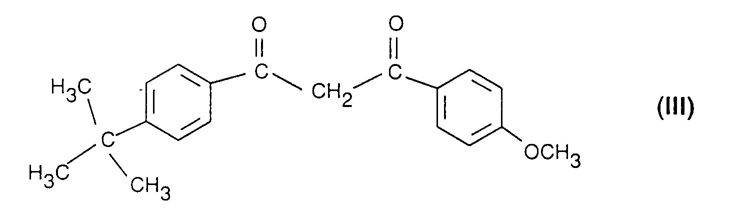

Le composé A (filtre UV-B à solubiliser) peut être présent dans les compositions selon l'invention à une concentration comprise entre 0,5 et 10 % en poids par rapport au poids total de la composition. Selon une caractéristique essentielle de la présente invention, ce composé doit se présenter dans la composition sous une forme totalement, ou substantiellement totalement, solubilisée.

Le composé B (solubilisant) peut être quant à lui présent dans les compositions selon l'invention à des teneurs comprises entre 2 et 15 % en poids par rapport au poids total de la composition. Selon une caractéristique essentielle des compositions selon l'invention, ce composé doit être utilisé en une quantité telle qu'elle soit suffisante pour solubiliser à elle seule la totalité, ou substantiellement la totalité, du composé A présent dans la composition.

Ainsi, la détermination des paramètres de solubilité effectuée par la Demanderesse (à température ambiante) du composé A dans le composé B montre que les conditions de solubilisation indiquées ci-dessus sont obtenues lorsque le rapport pondéral [(composé B)/(composé A)] est compris entre 0,3 et 30. De préférence, ce rapport est choisi, selon la présente invention, supérieur à 0,5 et inférieur à 25, encore plus préférentiellement inférieur à 10. A titre indicatif, la solubilité du composé A dans le composé B est de l'ordre de 60 % en poids.

Lorsque l'on désire en outre faire appel à un composé C (filtre UV-A solide) dans une composition conforme à l'invention, il convient alors que celui-ci soit également présent dans cette dernière sous une forme totalement, ou substantiellement totalement, solubilisée, en veillant, donc dans ce cas, soit d'utiliser une quantité de composé C qui reste compatible (c'est à dire capable de se dissoudre d'une part, et ceci, d'autre part, sans provoquer de déstabilisation ou tout autre perturbation) avec la phase constituée par le mélange [(composé A) + (composé B)] précédemment réalisé, soit d'utiliser initialement une quantité suffisante de composé B pour assurer une dissolution complète et stable du mélange [(composé A) + (composé C)]. A titre indicatif, il a été constaté qu'un mélange contenant un composé A et un composé C dans un rapport pondéral 3:1 est soluble à raison de près de 55 % en poids dans le composé B.

D'une manière générale, on notera que les concentrations en composés A, B et éventuellement C, sont choisies de manière telle que l'indice de protection solaire de la composition finale soit de préférence d'au moins 2.

Selon une caractéristique particulièrement avantageuse des compositions conformes à l'invention, ces dernières ne contiennent de préférence aucun, ou substantiellement aucun, solubilisant pour les composés A ou (A+C) autre que le composé B précédemment défini. Selon l'invention, on considère qu'un composé donné ne possède pas de propriétés solubilisantes vis à vis d'un autre composé donné lorsque ce dernier composé présente une solubilité inférieure à 1 % en poids environ dans ce premier composé.

En outre, toujours selon un mode préféré de réalisation de la présente invention, le support cosmétiquement acceptable dans lequel se trouvent contenus les différents composés A, B et éventuellement C, est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles autres, bien sûr, que les filtres lipophiles mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse représente généralement de 50 à 95 % en poids, de préférence de 70 à 90 % en poids, par rapport à l'ensemble de la formulation, la phase huileuse (comprenant les filtres lipophiles solubilisés et solubilisants) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation. On notera que, conformément à la présente invention, la phase grasse de telles émulsions peut n'être constituée pour l'essentiel ou même en totalité que du composé B (solvant organique) dans lequel se trouve(nt) solubilisé(s) le ou les filtres A et C précédemment définis, ainsi que les éventuels filtres complémentaires et autres adjuvants cosmétiques lipophiles classiques.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE 1

| *Emulsion huile-dans-eau* : | |
|---|---|
| - 4-méthylbenzylidène camphre ("EUSOLEX 6300") | 3 g |
| - α-cyano-β, β-diphénylacrylate de 2-éthylhéxyle ("UVINUL N539") | 6 g |
| - Mélange d'alcool cétylstéarylique et d'alcool cétylstéarylique oxyéthyléné à 33 moles d'oxyde d'éthylène vendu sous la dénomination "Sinnowax AO" par HENKEL (émulsionnant) | 7 g |
| - Mélange de mono- di et tristéarate de glycérol (co-émulsionnant) | 2 g |
| - Triglycérides d'acides gras en C8-C12 ("MIGLYOL 812") | 2 g |
| - Polydiméthylsiloxane | 1,5 g |
| - Alcool cétylique | 1,5 g |
| - Eau distillée | qsp 100g |

On réalise l'émulsion ci-dessus en dissolvant les filtres dans la phase grasse, puis en ajoutant les (co)émuisionnants dans cette phase grasse portée aux environs de 80°C, et enfin en additionnant sous agitation rapide l'eau préalablement chauffée à cette même température.

### EXEMPLE 2

| *Emulsion huile-dans-eau* : | |
|---|---|
| - 4-méthylbenzylidène camphre ("EUSOLEX 6300") | 4,5 g |
| - 4-(ter.-butyl) 4'-méthoxy dibenzoylméthane ("PARSOL 1789") | 1,5 g |
| - α-cyano-β, β-diphénylacrylate de 2-éthylhéxyle ("UVINUL N539") | 10 g |
| - Mélange d'alcool cétylstéarylique et d'alcool cétylstéarylique oxyéthyléné à 33 moles d'oxyde d'éthylène vendu sous la dénomination "Sinnowax AO" par HENKEL (émulsionnant) | 7 g |
| - Mélange de mono- di et tristéarate de glycérol (co-émulsionnant) | 2 g |
| - Triglycérides d'acides gras en C8-C12 ("MIGLYOL 812") | 2 g |
| - Polydiméthylsiloxane | 1,5 g |
| - Alcool cétylique | 1,5 g |
| - Eau distillée | qsp 100g |

Cette émulsion a été préparée comme à l'exemple 1.

### EXEMPLE 3

| *Emulsion eau-dans-huile* : | |
|---|---|
| - 4-méthylbenzylidène camphre ("EUSOLEX 6300") | 3 g |
| - 4-(ter.-butyl) 4'-méthoxy dibenzoylméthane ("PARSOL 1789") | 1 g |
| - α-cyano-β, β-diphénylacrylate de 2-éthylhéxyle ("UVINUL N539") | 8 g |
| - TiO₂ de qualité nanopigmentaire | 1,5 g |
| - Mélange [hydroxystéarate et isostéarate de sorbitol et de glycérol] à 20 moles d'oxyde de propylène et 30 moles d'oxydes d'éthylène, vendu sous le nom de "ARLACEL 780" par ICI | 2 g |
| - Conservateurs | qs |
| - Parfum | qs |
| - Eau | qsp 100g |

## Revendications

1. Compositions cosmétiques à usage topique, en particulier pour la photoprotection de la peau et/ou des cheveux, caractérisées par le fait qu'elles comprennent, dans un support cosmétiquement acceptable, (i) du 4-méthylbenzylidène camphre (composé A) sous forme solubilisée, à titre de filtre actif dans l'UV-B, et (ii) de l'α-cyano-β, β-diphénylacrylate de 2-éthylhéxyle (composé B) en une quantité suffisante pour solubiliser à lui seul la totalité dudit filtre UV-B.

2. Compositions selon la revendication 1, caractérisées par le fait que le composé A est présent à raison de 0,5 à 10 % en poids par rapport à l'ensemble de la composition.

3. Compositions selon l'une des revendications 1 ou 2, caractérisées par le fait que le composé B est présent à raison de 2 à 15 % en poids par rapport à l'ensemble de la composition.

4. Compositions selon l'une quelconque des revendications précédentes, caractérisées par le fait que le rapport pondéral [(composé B)/(composé A)] est compris entre 0,3 et 30.

5. Compositions selon la revendication 4, caractérisées par le fait que ledit rapport est supérieur à 0,5.

6. Compositions selon l'une des revendications 4 ou 5, caractérisées par le fait que ledit rapport est inférieur à 25, de préférence inférieur à 10.

7. Compositions selon l'une quelconque des revendications précédentes, caractérisées par le fait qu'elles comprennent en outre, sous une forme solubilisée, du 4-(ter.-butyl) 4'-méthoxy dibenzoylméthane (composé C) à titre de filtre actif dans l'UV-A.

8. Compositions selon l'une quelconque des revendications précédentes, caractérisées par le fait qu'elles sont exemptes ou substantiellement exemptes d'un agent solubilisant pour le composé A ou le mélange (composé A / composé C) autre que le composé B.

9. Compositions selon l'une quelconque des revendications précédentes, caractérisées par le fait que ledit support cosmétiquement acceptable se présente sous la forme d'une émulsion de type huile-dans-eau.

10. Compositions selon l'une quelconque des revendications précédentes, caractérisées par le fait qu'elles comprennent en outre un ou plusieurs filtres organiques complémentaires actifs dans l'UV-A et/ou UV-B, hydrophiles ou lipophiles.

11. Compositions selon la revendication 10, caractérisées par le fait que lesdits filtres organiques complémentaires sont choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres

12. Compositions selon l'une quelconque des revendications précédentes, caractérisées par le fait qu'elles comprennent en outre, à titre d'agents photoprotecteurs complémentaires, des pigments ou des nanopigments d'oxydes métalliques, enrobés ou non, capables de bloquer physiquement, par diffusion et/ou réflection, le rayonnement UV.

13. Compositions selon la revendication 12, caractérisées par le fait que lesdits pigments ou nanopigments sont choisis parmi les oxydes de titane, de zinc, de fer, de zirconium, de cérium et leurs mélanges, enrobés ou non.

14. Compositions selon l'une quelconque des revendications précédentes, caractérisées par le fait qu'elles comprennent en outre au moins un agent de bronzage et/ou de brunissage artificiel de la peau.

15. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

16. Compositions selon l'une quelconque des revendications précédentes, caractérisées par le fait qu'il s'agit de compositions protectices de l'épiderme humain ou de compositions antisolaires et qu'elles se présentent sous forme de dispersions vésiculaires non ioniques, émulsions, en particulier émulsions de type huile-dans-eau, crèmes, laits, gels, gels crèmes, suspensions, dispersions, poudres, bâtonnets solides, mousses ou spray.

17. Compositions selon l'une quelconque des revendications 1 à 15, caractérisées par le fait qu'il s'agit de compositions de maquillage des cils, des sourcils ou de la peau et qu'elles se présentent sous forme solide ou pâteuse, anhydre ou aqueuse, d'émulsions, de suspensions ou de dispersions.

18. Compositions selon l'une quelconque des revendications 1 à 15, caractérisées par le fait qu'il s'agit de compositions destinées à la protection des cheveux contre les rayons ultraviolets et qu'elles se présentent sous forme de shampooings, de lotions, de gels, d'émulsions, de dispersions vésiculaires non ioniques ou de laques pour cheveux.

19. Compositions selon l'une quelconque des revendications précédentes, caractérisées par le fait qu'elles présentent un indice de protection sur peau d'au moins 2.

20. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

21. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 19.

22. Utilisation de l'α-cyano-β, β-diphénylacrylate de 2-éthylhéxyle pour solubiliser du 4-méthylbenzylidène camphre ou un mélange de 4-méthylbenzylidène camphre et de 4-(ter.-butyl) 4'-méthoxy dibenzoylméthane, dans une composition cosmétique antisolaire.

## Claims

1. Cosmetic compositions for topical use, in particular for the photoprotection of the skin and/or the hair, characterized in that they comprise, in a cosmetically acceptable support, (i) 4-methyl-benzylidenecamphor (compound A) in dissolved form, as screening agent active in the UV-B range, and (ii) 2-ethylhexyl α-cyano-β,β-diphenylacrylate (compound B) in an amount which is sufficient to dissolve by itself all of the said UV-B screening agent.

2. Compositions according to Claim 1, characterized in that compound A is present in a proportion of from 0.5 to 10% by weight relative to the whole composition.

3. Compositions according to either of Claims 1 and 2, characterized in that compound B is present in a proportion of from 2 to 15% by weight relative to the whole composition.

4. Compositions according to any one of the preceding claims, characterized in that the [(compound B)/(compound A)] weight ratio is between 0.3 and 30.

5. Compositions according to Claim 4, characterized in that the said ratio is greater than 0.5.

6. Compositions according to either of Claims 4 and 5, characterized in that the said ratio is less than 25, preferably less than 10.

7. Compositions according to any one of the preceding claims, characterized in that they also comprise, in dissolved form, 4-(tert-butyl)-4'-methoxydibenzoylmethane (compound C) as screening agent active in the UV-A range.

8. Compositions according to any one of the preceding claims, characterized in that they are free or substantially free of an agent for dissolving compound A or the mixture (compound A/compound C) other than compound B.

9. Compositions according to any one of the preceding claims, characterized in that the said cosmetically acceptable support is in the form of an emulsion of oil-in-water type.

10. Compositions according to any one of the preceding claims, characterized in that they also comprise one or more hydrophilic or lipophilic complementary organic screening agents active in the UV-A and/or UV-B range.

11. Compositions according to Claim 10, characterized in that the said complementary organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives, screening polymers and screening silicones.

12. Compositions according to any one of the preceding claims, characterized in that they also comprise, as complementary photoprotective agents, coated or uncoated metal oxide pigments or nanopigments, capable of physically blocking out the UV radiation by scattering and/or reflection.

13. Compositions according to Claim 12, characterized in that the said pigments or nanopigments are chosen from titanium oxide, zinc oxide, iron oxide, zirconium oxide, cerium oxide and mixtures thereof, which may be coated or uncoated.

14. Compositions according to any one of the preceding claims, characterized in that they also comprise at least one agent for artificially tanning and/or browning the skin.

15. Compositions according to any one of the preceding claims, characterized in that they also comprise at least one adjuvant chosen from fatty substances, organic solvents, ionic or nonionic thickeners, softeners, antioxidants, opacifiers, stabilizers, emollients, silicones, α-hydroxy acids, antifoaming agents, moisturizers, vitamins, fragrances, preserving agents, surfactants, fillers, sequestering agents, polymers, propellants, basifying or acidifying agents and dyes.

16. Compositions according to any one of the preceding claims, characterized in that they are compositions for protecting the human epidermis or antisun compositions and in that they are in the form of nonionic vesicle dispersions, emulsions, in particular emulsions of oil-in-water type, creams, milks, gels, cream-gels, suspensions, dispersions, powders, solid tubes, mousses or sprays.

17. Compositions according to any one of Claims 1 to 15, characterized in that they are compositions for making up the eyelashes, the eyebrows or the skin and in that they are in solid or pasty, anhydrous or aqueous form or in the form of emulsions, suspensions or dispersions.

18. Compositions according to any one of Claims 1 to 15, characterized in that they are compositions intended for protecting the hair against ultraviolet rays and in that they are in the form of shampoos, lotions, gels, emulsions, nonionic vesicle dispersions or lacquers for the hair.

19. Compositions according to any one of the preceding claims, characterized in that they have a protection factor on the skin of at least 2.

20. Use of the compositions defined in any one of the preceding claims as, or for the manufacture of, cosmetic compositions for protecting the skin and/or the hair against ultraviolet radiation, in particular solar radiation.

21. Cosmetic treatment process for protecting the skin and/or the hair against ultraviolet radiation, in particular solar radiation, characterized in that it consists in applying an effective amount of a composition as defined in any one of Claims 1 to 19 to the skin and/or the hair.

22. Use of 2-ethylhexyl α-cyano-β,β-diphenylacrylate to dissolve 4-methylbenzylidenecamphor or a mixture of 4-methylbenzylidenecamphor and 4-(tert-butyl)-4'-methoxydibenzoylmethane, in an antisun cosmetic composition.

## Patentansprüche

1. Kosmetische Zusammensetzungen zur topischen Anwendung, insbesondere zum Lichtschutz der Haut und/oder der Haare, dadurch gekennzeichnet, daß sie in einem kosmetisch akzeptablen Träger (i) 4-Methyl-benzylidencampher (Verbindung A) in solubilisierter Form als im UV-B-Bereich wirksames Filter und (ii) 2-Ethylhexyl-α-cyano-β,β-diphenylacrylat (Verbindung B) in einer Menge, die allein ausreicht, um das gesamte UV-B-Filter zu solubilisieren, enthalten.

2. Zusammensetzungen nach Anspruch 1, dadurch gekennzeichnet, daß die Verbindung A in einem Anteil von 0,5 bis 10 Gew.-%, bezogen auf die gesamte Zusammensetzung, vorliegt.

3. Zusammensetzungen nach einem der Ansprüche 1 oder 2, dadurch gekennzeichnet, daß die Verbindung B in einem Anteil von 2 bis 15 Gew.-%, bezogen auf die gesamte Zusammensetzung, vorliegt.

4. Zusammensetzungen nach einem der vorhergehenden Ansprüche, dadurch gekennzeichnet, daß das Gewichtsverhältnis [(Verbindung B) / (Verbindung A)] im Bereich von 0,3 bis 30 liegt.

5. Zusammensetzungen nach Anspruch 4, dadurch gekennzeichnet, daß dieses Verhältnis über 0,5 liegt.

6. Zusammensetzungen nach einem der Ansprüche 4 oder 5, dadurch gekennzeichnet, daß dieses Verhältnis unter 25 und vorzugsweise unter 10 liegt.

7. Zusammensetzungen nach einem der vorhergehenden Ansprüche, dadurch gekennzeichnet, daß sie ferner 4-(*tert*.-Butyl)-4'-methoxy-dibenzoylmethan (Verbindung C) in solubilisierter Form als im UV-A-Bereich wirksames Filter enthalten.

8. Zusammensetzungen nach einem der vorhergehenden Ansprüche, dadurch gekennzeichnet, daß sie frei oder im wesentlichen frei von solubilisierenden Mitteln für die Verbindung A oder das Gemisch (Verbindung A / Verbindung C) sind, die von der Verbindung B verschieden sind.

9. Zusammensetzungen nach einem der vorhergehenden Ansprüche, dadurch gekennzeichnet, daß der kosmetisch akzeptable Träger in Form einer Öl-in-Wasser-Emulsion vorliegt.

10. Zusammensetzungen nach einem der vorhergehenden Ansprüche, dadurch gekennzeichnet, daß sie ferner ein oder mehrere ergänzende hydrophile oder lipophile organische Filter enthalten, die im UV-A-Bereich und/oder UV-B-Bereich wirksam sind.

11. Zusammensetzungen nach Anspruch 10, dadurch gekennzeichnet, daß die ergänzenden organischen Filter ausgewählt sind unter Zimtsäurederivaten, Salicylsäurederivaten, Campherderivaten, Triazinderivaten, Benzophenonderivaten, Dibenzoylmethanderivaten, β,β-Diphenylacrylatderivaten, p-Aminobenzoesäurederivaten, polymeren Filtern und Siliconfiltern.

12. Zusammensetzungen nach einem der vorhergehenden Ansprüche, dadurch gekennzeichnet, daß sie ferner als ergänzende Lichtschutzmittel gegebenenfalls umhüllte Pigmente oder Nanopigmente von Metalloxiden enthalten, die die UV-Strahlung physikalisch durch Streuung und/oder Reflexion zu sperren vermögen.

13. Zusammensetzungen nach Anspruch 12, dadurch gekennzeichnet, daß diese Pigmente oder Nanopigmente ausgewählt sind unter gegebenenfalls umhüllten Oxiden von Titan, Zink, Eisen, Zirconium, Cer und deren Gemischen.

14. Zusammensetzungen nach einem der vorhergehenden Ansprüche, dadurch gekennzeichnet, daß sie außerdem mindestens ein Bräunungsmittel und/oder ein Mittel zur künstlichen Hautbräunung enthalten.

15. Zusammensetzungen nach einem der vorhergehenden Ansprüche, dadurch gekennzeichnet, daß sie ferner mindestens einen Zusatzstoff enthalten, der ausgewählt ist unter Fettsubstanzen, organischen Lösemitteln, ionischen oder nichtionischen Verdickungsmitteln, reizlindernden Mitteln, Antioxidantien, Trübungsmitteln, Stabilisierungsmitteln, Emollentien, Siliconen, α-Hydroxysäuren, Mitteln gegen Schaumbildung, Hydratisierungsmitteln, Vitaminen, Parfums, Konservierungsmitteln, grenzflächenaktiven Stoffen, Füllstoffen, Maskierungsmitteln, Polymeren, Treibmitteln, Mitteln zum Alkalischmachen oder zum Ansäuern und Färbemitteln.

16. Zusammensetzungen nach einem der vorhergehenden Ansprüche, dadurch gekennzeichnet, daß es sich um Zusammensetzungen zum Schutz der menschlichen Epidermis oder um Sonnenschutzzusammensetzungen handelt und daß sie in Form von nichtionischen Vesikeldispersionen, Emulsionen, insbesondere Öl-in-Wasser-Emulsionen, Cremes, Milchen, Gelen, Gel-Cremes, Suspensionen, Dispersionen, Pulvern, festen Stiften, Schäumen oder Sprays vorliegen.

17. Zusammensetzungen nach einem der Ansprüche 1 bis 15, dadurch gekennzeichnet, daß es sich um Zusammensetzungen zum Schminken der Wimpern, der Augenbrauen oder der Haut handelt und daß sie in fester oder pastöser, wasserfreier oder wässeriger Form, als Emulsionen, Suspensionen oder Dispersionen vorliegen.

18. Zusammensetzungen nach einem der Ansprüche 1 bis 15, dadurch gekennzeichnet, daß es sich um Zusammensetzungen handelt, die für den Schutz der Haare gegen ultraviolette Strahlung bestimmt sind, und daß sie in Form von Haarwaschmitteln, Lotionen, Gelen, Emulsionen, nichtionischen Vesikeldispersionen oder Haarlacken vorliegen.

19. Zusammensetzungen nach einem der vorhergehenden Ansprüche, dadurch gekennzeichnet, daß sie einen Lichtschutzfaktor auf der Haut von mindestens 2 aufweisen.

20. Verwendung von in einem der vorhergehenden Ansprüche definierten Zusammensetzungen als kosmetische Zusammensetzungen zum Schutz der Haut und/oder der Haare gegen ultraviolette Strahlung und insbesondere gegen Sonnenlicht, oder zur deren Herstellung.

21. Verfahren zur kosmetischen Behandlung zum Schutz der Haut und/oder der Haare gegen ultraviolette Strahlung und insbesondere gegen Sonnenlicht, dadurch gekennzeichnet, daß es darin besteht, eine wirksame Menge einer in einem der Ansprüche 1 bis 19 definierten Zusammensetzung auf die Haut und/oder die Haare aufzutragen.

22. Verwendung von 2-Ethylhexyl-α-cyano-β,β-diphenylacrylat zur Solubilisierung von 4-Methyl-benzylidencampher oder eines Gemisches von 4-Methyl-benzylidencampher und 4-(*tert*.-Butyl)-4'-methoxy-dibenzoylmethan in einer kosmetischen Sonnenschutzzusammensetzung.
